Europäisches Patentamt

European Patent Office

Office européen des brevets

(11) Publication number: **0 230 864**
**A1**

(12) **EUROPEAN PATENT APPLICATION**

(21) Application number: 86830025.2

(22) Date of filing: 31.01.86

(51) Int. Cl.³: **A 61 L 2/18**
**A 61 M 1/28**

(43) Date of publication of application:
05.08.87 Bulletin 87/32

(84) Designated Contracting States:
AT BE CH DE FR GB LI NL SE

(71) Applicant: Buoncristiani, Vincenzo
Via Marco Polo 4
I-06034 Foligno (Perugia)(IT)

(72) Inventor: Buoncristiani, Vincenzo
Via Marco Polo 4
I-06034 Foligno (Perugia)(IT)

(74) Representative: Mascioli, Alessandro, Prof.Dr.
c/o A.N.D.I. Associazione Nazionale degli Inventori Via
Lima, 35
I-00198 Roma(IT)

(54) **Circuit for sterilising ducts and connection elements between two containers for the effusion of sterilised liquids.**

(57) A device for keeping the initial sterilization of liquids during the passing thereof from a container X to a container Y, characterized in that the sterilization takes place during the functioning of ducts A and E communicating between the two containers, of the connection elements B and D, and that said connection elements will form a definite conjunction, always keeping the same in a sterilization chamber C, and therefore in an aseptic place.

Fig . 2.

EP 0 230 864 A1

Circuit  for sterilizing ducts and connection ele =
ments between two containers for the effusion of ste=
rilized liquids.

VINCENZO BUONCRISTIANI - ITALY

The present invention concerns a device for steriliz
ing, by means of a disinfectant, ducts and connection
elements used between two containers for the effusion,
from one to the other, of sterilized liquid, maintain
ing the original sterilization thereof.

The problem of the sterilization of the ducts and of
the connection elements placed between two containers
for the effusion of sterilized liquids, is of enor =
mous importance, because the alteration of the origi
nal sterilization may cause the development of bacte
ria or similar which in turn may provoke serious  infections in those who use said liquids, which are
particularly used in medical fields.

One of the possible fields in which it may result to
be extremely important to guarantee a perfect steri=
lization is the continuous ambulatorial peritonaeum
dialysis (CAPD).

It is already well known that the peritonaeum dialy=
sis is a technique used to free the patient's orga =
nism from toxic substances it may have accumulated
for different reasons: mainly the insufficient func=
tioning of the kidneys.

Said technique consists in introducing into the abdo
minal cavity a solution of appropriate composition
and quantity and taking it off after said solution
has absorbed the toxic substances to be eliminated
by means of diffusion with the blood through the pe=
ritonaeum membrane and the internal organs comprised
therein.

Said cleaning cycle must be repeated in proportion
to the need of depuration shown by the single patient.

The main application limit of said therapeutic te=
chnique - which is easy to perform and rather effi=
cacious - is given by the high number of peritonitis
which it causes due to the introduction of germs into
the abdominal cavity by the eventual infection at
the connection point between the solution defluction
tube and the catheter leading the solution into the
abdomen.

This kind of inconvenience happens with a particular
frequency in the use of the new continuous ambulato=
rial peritonaeum dialysis (CAPD), due to the high con
nection number between the catheter and the contain=
er of the solution requested by this technique.

Various systems have been tried to eliminate the la=
tent danger of infections, comprising the one suggest
ed by Oreopulos and the one called "Perugia CAPD Sy= stem". Even if, by use of these systems, the number of peritonitis cases has been rather reduced, this kind of infection heavily weighs on said technique and limits the use thereof.

It is the aim of the present invention to allow a perfect sterilization of any system of sterilized liquid effusion, with a particular attention to the application thereof to the continuous ambulatorial peritonaeum dialysis.

The present invention comprises, also relating to the attached claims: a sterilization chamber in which the ends of the ducts to be connected are introduced and sterilized by means of a disinfectant without taking said ends off, and therefore always keeping them in an aseptic place; a perforable closing mem= brane of one of the ducts to be connected; a perfo= rating connector applied to one of the ducts to be connected; an autostopping device to be coupled and fixed to the main body and a coupling device between the ducts and the sterilization chamber consisting in two valves united by a hinge.

The present invention will be described in detail ac cording to the attached drawings in which some pre = ferred embodiments are shown.

Figure 1 shows a scheme of the coupling of containers X and Y.

Figures 2 and 3 respectively show a vertical and an exploded section of another embodiment with a three-way connection, which may be applied in the perito= naeum dialysis.

Figure 4 shows a plan view of the detail of the coup ling device in the position with open valves.

The figures show a sterilization device comprising:
- ducts A and A' projecting from containers X and Y;
- sterilization chambers C and C' which may always be connected to ducts E and E';
- a communication circuit between chambers C and C' consisting in ducts E and E' joining in duct F through branch H;
- ducts G and G' both respectively deriving from cham bers C and C';
- opening and closing devices I, I', L and M, which may be operated from the outside by deflection, of the ducts onto which they are placed;
- a device for respectively coupling ducts A and A' to C and C';
- ends B, D, B' and D' so as to allow an easy and quick connection between B and D and B' and D';
- a device for moving, from the outside, B towards D and B' towards D', or viceversa D towards B and D' towards B', which movement is necessary to defini= tely connect ducts A and E and A' and E' and thus placing into communication containers X and Y;

- a closing device applied onto ends B and B' so as to allow, due to the form and structure thereof:

a) the sterilization of the whole outer surface which will get into contact with D and D' by means of connection operations;

b) the opening, by means of the connection opera= tions, between A and E and between A' and E', of the communication between containers X and Y.

According to the present invention and provided that a sterilized liquid shall be effused from container X to container Y, also sterilized, and so as to avoid the contamination of said liquid, the following steps must be followed:

1) ducts A and A' shall be fixed to respective steri_ lization chambers C and C', so that ends B and B' may project inside said chambers;

2) provided that chambers C and C' are permanently connected to respective ducts E and E', said cham_ bers will be fixed in a vertical position so that B and B' are placed in the high part;

3) by means of devices I, I', L and M the ducts, on which said devices are placed, will be opened;

4) through duct F the sterilization substance will be introduced up to a level to cover B and B', avoid_ ing the forming of air bubbles in the whole transfer circuit, comprising chambers C and C' (the air will find an outlet through G and G') and then L and M will be closed;

5) when the time necessary for the sterilization has passed, the connection between B and D will be effected, so that the connection between A and E can also be performed;

6) L will be opened and the sterilized liquid contained in X will flow down to effect the washing of ducts E and F so as to totally expell the sterilizing substance; when this is done, L will be closed and M opened; now the sterilized liquid will flow through the other branch of the circuit, expelling the sterilization substance from E' and C' through G' and effecting the necessary washing: at this point B' and D' are connected.

With this last operation containers X and Y are placed in communication and the effusion of the liquid can be performed, maintaining the original sterilization.

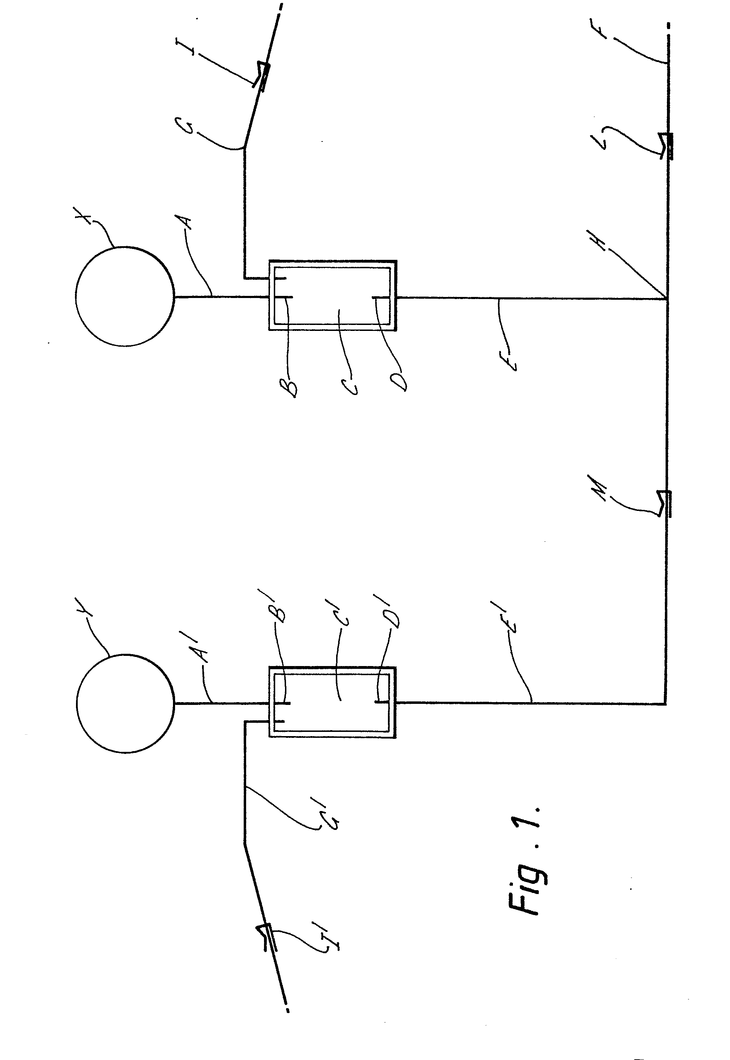

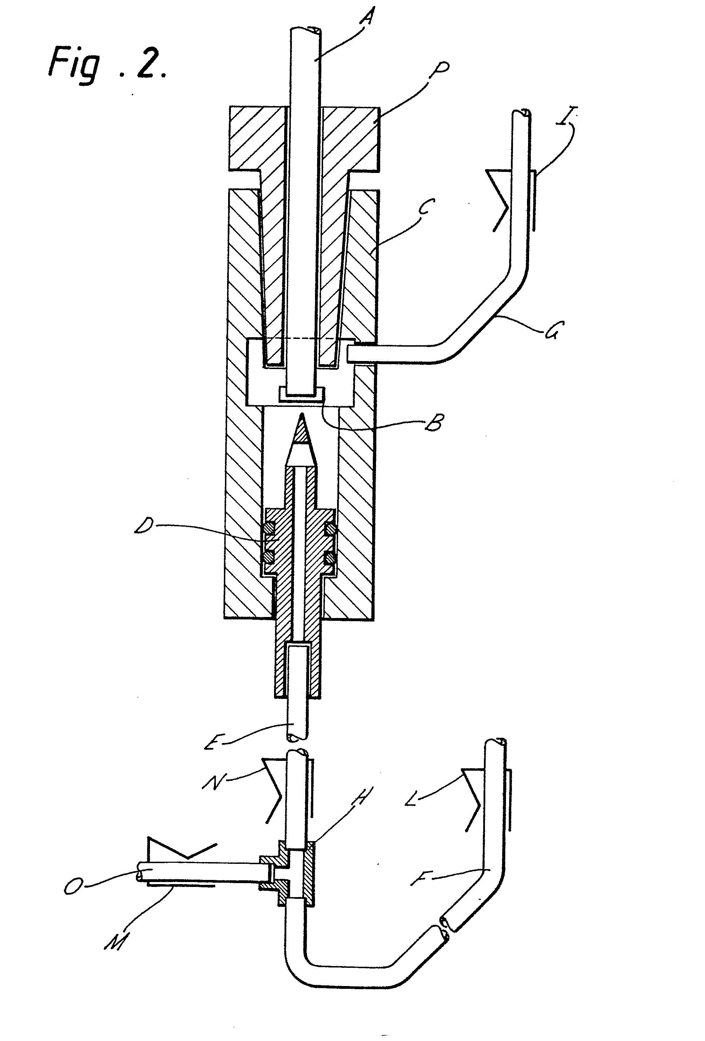

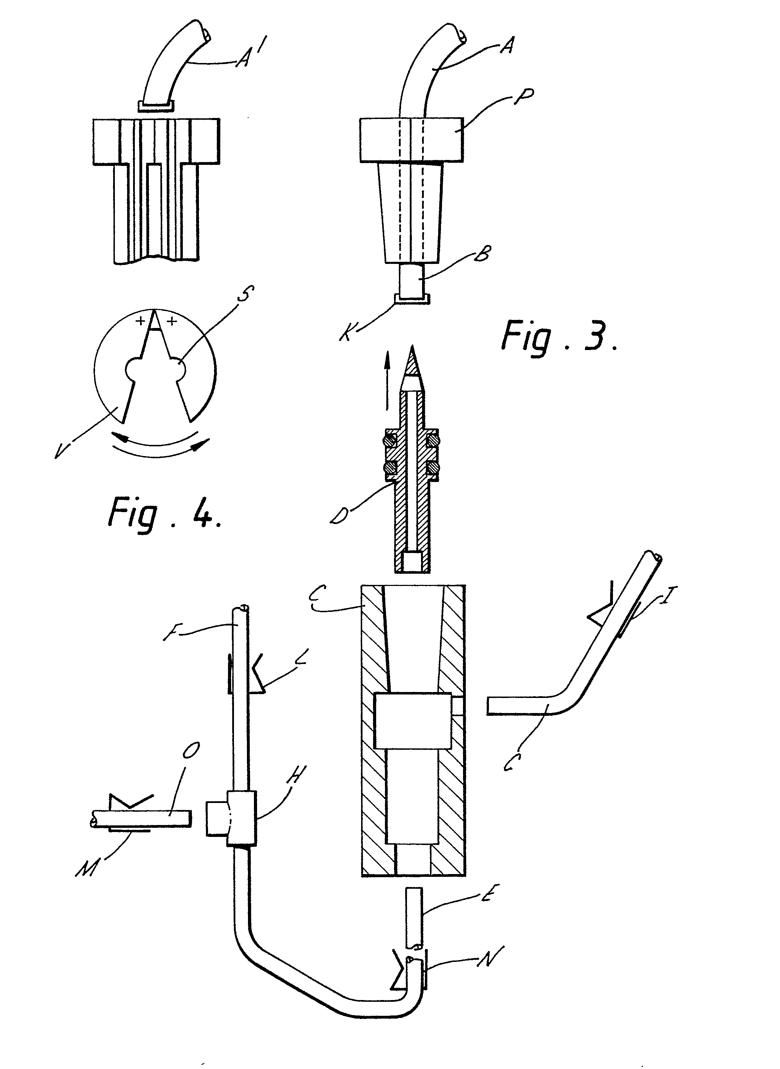

For what concerns the application of the present invention in the continuous ambulatorial peritonaeum dialysis, according to figures 2, 3 and 4, the device comprises the use of only one part of the scheme of figure 1, as container Y is now replaced by the abdominal cavity and therefore all that part shown by E', D', C', B', A', G', I' and Y will be eliminated.

Furthermore, branch H of figure 1 must be replaced by a three-way connection which, beyond connecting ducts E and F, shows a third way for the closing with a cap or as well for the connection with duct O, provided with device M and placed like a prothesis onto the catheter and with the free end for the closing thereof with a cap or for the connection with the three-way connection; further, the addition of closing and opening device N is provided.

Referring to the details of another embodiment according to figures 2, 3 and 4, they show:
- device P for coupling A with C, autostopping due to its conicity, consisting in two valves V united by a hinge; said device, when extracted from the seat, can be opened and duct A can be introduced into the groove thereof;
- the closing of duct A, consisting in perforable membrane K placed, like a hat, on end B of said duct;
- the main body of the whole device, consisting in transparent material, having a longitudinal cavity and provided, at one side, for the coupling and locking with P, while on the other side the perforating connector, coaxial to duct A contained in P, is placed; in the central part of said main body remains a free space, corresponding to the sterilization chamber;

- perforating connector D sliding in the main body and perfectly tight with respect to the same; a projection of said connector to which duct E is connected is of such dimension and form that it can be handled from outside.

For what concerns the functioning of the invention in the embodiment for the continuous ambulatorial peritonaeum dialysis (some days after placing of the catheter in the abdominal cavity, when the abdomen of the patient is full with solution), prothesis O will be connected to the catheter, said prothesis carrying a cap at the opposite end and with closing system M locked.

Once removed the cap from the free end of the prothesis, the same will be filled up with the disinfectant and then closed again. The fresh solution pocket is then connected to the transfer circuit by introducing the end part of duct A into the valve coupling device P pocket, which in turn will be introduced into the apposite seat of the upper part of the main body containing the sterilization chamber C.

Further, the free end of the prothesis will be con= nected to the third way of connection H of the trans fer circuit. Once L is opened through F, the circuit gets filled up with disinfectant, taking great care in eliminating the air bubbles from connection H as well as from the sterilization chamber, making them flow down through duct G.

Now L is closed and it is attended until the disin= fectant has performed its action. Then ducts A are connected and membrane B is perforated by the advanc ing of point D. Once L has been opened a certain am= ount of fresh solution is made to flow down thus washing the whole circuit removing the disinfec tant contained therein.

After a determined quantity of solution has washed the circuit (about 100 ml.), N is closed, M is open ed and the solution contained in the abdomen and which is now saturated with toxins from the blood, flows down.

The outflow of said solution has also the task of completely removing the disinfectant from prothesis O.

Once the outflow from the abdomen has ended, N will be opened again and L closed, thus filling up the abdomen with the fresh solution. Once the filling up is ended, M closes the inlet to the peritonaeum cavi ty and after L is opened, the transfer circuit will be filled up with the disinfectant until it has filled the pocket outflow of duct A.

The same duct will be closed by the system of tighten ing the tubes and the duct will be cut above the clos ing. When L and N are also closed, prothesis O will be detached from connection H, which will be closed with the apposite cap. Now prothesis O will be filled up with the disinfectant; finally, the inlet to O will be closed with the apposite cap and the patient can freely move about until the next effusion, without any limitation and without any need of carrying along the transfer set and the usually used pocket and, fur thermore, the prothesis will be always protected by the disinfectant.

At the time of the following effusion it will be suf ficient, as the prothesis is already placed and full of disinfectant, to repeat the cycle.

CLAIMS

1. Circuit for the sterilization of ducts and the connective elements between a container and a receiver for the effusion of sterilized liquids therebetween comprising:

a frist duct (A) having a first end connected to the container (X) and having a second end;

a chamber (C) having first and second openings;

a valve member (P) including first and second valve halves hingedly connected together and capable of assuming a closed position in which said valve member has a groove therein, and an open position, said valve in said open position adapted to receive said second end of said first duct within said groove, said valve in the closed position adapted to retain said second end of said first duct in said groove for the insertion with said first opening of said chamber (C) to permit liquid flow between the interior of said chamber (C) and the container (X) connected to said first duct;

a second duct (E) having a first end (D) received by said second opening of chamber (C) and having a second opening adapted for the connection to the receiver, said first end (D) of said second duct (E) including a perforating member movable between a non operative position and an operative position, said second end (B) of said first duct (A) further being closed by a membrane capable of being perforated by said perforating member to cennect the duct (A) and the duct (E) within said chamber (C) when said perforating member is moved to said operative position;

a connector member (H);

a third duct (F), having a first end connected to said second duct through said connector member (H);

a fourth duct (G) having a first end connected to said chamber for conducting air out of said chamber (C);

a first valve (I) for opening or closing said fourth duct;

a second valve (L) for opening or closing said third duct (F);

a fourth valve (N) for opening or closing said second duct (E).

2. Circuit as claimed in claim 1 wherein said circuit further comprises a prothesis (O), selectively closable in (M), having a first end adapted for connecting with a catheter communicating with the abdominal cavity of a patient and having a second end adapted to connect to the second end of duct (E), and the third valve (M) being disposed to contol fluid flow between said prothesis (O) and said connector (H).

3. Circuit as claimed in claim 2 wherein said fourth valve (N) selectively controls the fluid flow between said second duct (E) and said connector member (H).

4. Method for performing a peritoneum dialysis using the circuit as claimed in claim 2 comprising the steps of:

closing said third valve (M) and connecting said prothesis (O) to the catheter on the patient;

opening said prothesis (O) to fill the same with disinfectant;

introducing said second end of said first duct (A) into said valve member (P) in the open position and closing said valve halves to retain said duct end therebetween;

introducing said valve member (P) into said first opening of said chamber (C);

opening said second valve (L) to fill said circuit with disinfectant;

opening said first valve (I) for exhausting air bubbles introduced into said circuit by the disinfectant;

closing said second valve (L);

moving said perforating member to perforate said membrane and thereby communicatively connect said first duct (A) and said second duct (E);

opening said second valve (L) so that the disinfectant is washed from said circuit by dialysis solution;

closing said fourth valve (N);

opening said third valve (M) to permit used dialysis solution to exit the patient's abdomen, the used solution further washes disinfectant from said prothesis;

opeing said fourth valve (N) and closing said second valve (L) for filling the abdomen with fresh dialysis solution;

closing said third valve (M) to isolate the abdomen cavity from said circuit;

opening said second valve (L) to refill said circuit with disinfectant;

severing said first duct (A) at about just above said valve member ;

detaching said prothesis (O) from said connector member (H) and filling the prothesis with disinfectant;

closing said prothesis (O) to confine the disinfectant therein.

5. Circuit for transferring sterile fluids between a container (X) and a receiver (Y) comprising:

a first duct (A) having a first end for receiving the fluid from the container (X) and having a second end (B);

a sterilization chamber (C) having a first opening adapetd to receive said second end (B) of said first duct (A) therethrough and having a second opening;

a second duct (E) having a first end (D) adapted to be received by said second opening of said chamber (C) and to thereby communicate with said first duct (A) within said chamber (C), and having a second end adapted for communication with the receiver;

a third duct (F) selectively communicating with the second duct (E) for introducing the disinfectant solution, to fill said circuit and discharging the disinfectant from said circuit;

valving means (L) for selectively introducing the disinfectant into said circuit through said third duct (F) to fill the circuit, for introducing the sterile fluid into the circuit to flush the disinfectant out of the circuit and for conducting the sterile fluid to the receiver (Y) through said first and second ducts.

6. Circuit as claimed in claim 5 further comprising a fourth duct (G), connected to said chamber (C) for selectively conducting air out of said chamber (C).

7. Circuit as claimed in claim 6 wherein one of said (A) duct ends includes a perforable membrane and the other includes a perforating member, said perforating member being movable from an inoperative position to an operative position wherein said member perforates said membrane to produce fluid communication between said first (A) and second (E) ducts.

8. Circuit as claimed in claim 7 further comprising a first valve (I) for opening or closing said fourth duct (G); a second valve (L) for opening or closing said third duct (F); a fourth valve (N) for opening or closing said second duct.

9. Circuit as claimed in claim 8 comprising a three-way connective element (H) including a first end connected to said second end of said second duct (E), a second end connected to said third duct (F) and a third end adapted for the connection with a catheter (O).

10. Circuit as claimed in claim 9 further comprising a prothesis for connecting said third end of said connective element (H) to the catheter (O) and a fourth valve (N) for controlling fluid flow between said second duct (E) and said connective element.

0230864

Fig . 1.

0230864

Fig . 2.

A'

A

P

B

K

Fig . 3.

S

+  +

V

Fig . 4.

D

C

I

F

L

C

O

H

M

E

N

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | WO-A-8 302 395 (BAXTER TRAVENOL LABORATORIES, INC.) * claim 1; page 3, line 28 - page 4, line 2; figures 1-5 * | 1 | A 61 L 2/18<br>A 61 M 1/28 |
| A | EP-A-0 049 525 (SIS-TER S.P.A.) * claim 13; figures 1, 2 * | 1 | |
| A | EP-A-0 029 526 (S.P.A. "S.I.F.R.A.") * claim 1 * | 5 | |
| A | FR-A-2 367 516 (GAMBRO AB) * claims 1, 3, 4, 7; figure * | 1,5 | |

TECHNICAL FIELDS SEARCHED (Int. Cl 4)

A 61 L 2/00
A 61 M 1/00
A 61 M 25/00

The present search report has been drawn up for all claims

| Place of search | Date of completion of the search | Examiner |
|---|---|---|
| BERLIN | 20-03-1987 | PAPA E.R. |